# FASCICULE DE BREVET EUROPEEN

(11) **EP 0 731 666 B1**
(45) Date de publication et mention de la délivrance du brevet: **23.05.2001**
(21) Numéro de dépôt: 95932055.7
(22) Date de dépôt: 26.09.1995
(51) Int. Cl.: A61B 5/087

(54) **APPAREIL DE MESURE DE DEBIT EXPIRATOIRE DE POINTE**
VORRICHTUNG ZUR MESSUNG DER MAXIMALSTRÖMUNG DER AUSATEMLUFT
APPARATUS FOR MEASURING THE PEAK EXPIRATORY RATE

(30) Priorité: 30.09.1994 FR 9411976
(43) Date de publication de la demande: 18.09.1996
(73) Titulaire: SOCIETE DE THERMOFORMAGE ET D'INJECTION DES PLASTIQUES- S.T.I. PLASTIC, 38160 Beauvoir-en-Royans (FR)
(72) Inventeur: MOULIN, Jacques, F-38160 Saint-Romans (FR)
(74) Mandataire: de Beaumont, Michel
(86) Numéro de dépôt international: FR9501234
(87) Numéro de publication internationale: WO9610360

(56) Documents cités:
- EP-A- 0 295 575
- US-A- 3 608 546
- US-A- 5 137 026

## Description

La présente invention concerne un appareil de mesure de caractéristiques pulmonaires d'un patient. Plus particulièrement, elle vise un spiromètre portable pour mesurer divers paramètres nécessaires au diagnostic des médecins, tels que le débit expiratoire de pointe, le VEMS (Volume Expiratoire Maximal par Seconde), etc.

La mesure du débit expiratoire maximal est souvent effectuée pour les personnes asthmatiques, bronchopathes, atteintes de mucoviscidose ou les transplantés pulmonaires. En effet, notamment pour les asthmatiques, cette mesure permet d'anticiper la survenance de crises d'asthme et, en cours de traitement, elle aide à pondérer la prise de médicaments.

Il est donc souhaitable que des patients puissent analyser régulièrement leur débit expiratoire de pointe, fassent des relevés réguliers de leurs caractéristiques et puissent les communiquer à leur médecin ou les utiliser directement d'une façon indiquée par leur médecin.

Il existe des spiromètres de type mécanique dans lesquels le souffle d'un patient agit à l'encontre d'un piston rappelé par un ressort. Ces appareils mécaniques présentent l'avantage d'être peu coûteux et de faible encombrement mais présentent l'inconvénient d'être peu fiables, peu précis et peu fidèles.

Pour réaliser un appareil de mesure de débit électronique, il est connu d'utiliser un capteur de débit à Venturi. Un tel capteur comprend un tube muni d'un retrécissement. Lorsqu'un patient souffle dans le tube, il s'établit une différence de pression de part et d'autre du rétrécissement qui est sensiblement proportionnelle au débit dans une certaine plage de mesure selon les dimensions et la forme du rétrécissement. Cette différence est mesurée par un capteur de pression différentiel relié à des régions du tube situées de part et d'autre du rétrécissement par des conduits.

Un problème spécifique qui se pose dans l'utilisation d'un capteur de débit à Venturi dans un spiromètre réside dans le fait qu'il faut que le spiromètre soit susceptible d'être nettoyé et stérilisé régulièrement, éventuellement après chaque usage. En particulier, il faut éviter que des germes, provenant de la salive ou d'autres expectorations du patient, puissent proliférer dans le tube et dans les conduits qui mènent au capteur. De plus, le nettoyage n'est pas aisé à cause du passage fonctionnel relativement étroit au niveau du rétrécissement et à cause d'angles rentrants inévitables qui sont difficiles à atteindre et qui accumulent des salissures.

Le brevet américain 5 137 026 décrit un spiromètre électronique portable utilisant un tel capteur de débit à Venturi. Le tube de mesure est solidaire du boîtier de l'appareil. Pour éviter d'encrasser les conduits qui mènent au capteur de pression, des filtres particuliers sont disposés dans ces conduits à l'endroit où ils débouchent dans le tube. Ces filtres doivent être étanches au liquide et perméables à l'air. Pour nettoyer et stériliser le tube, il faut tremper l'appareil entier dans une solution antiseptique. Pour cela, l'appareil doit être parfaitement étanche, ce qui augmente notablement son coût.

Un objet de la présente invention est de prévoir un spiromètre électronique portable ne présentant pas de problèmes de nettoyage et de stérilisation.

Un autre objet de la présente invention est de prévoir un tel spiromètre qui soit de faible encombrement et facile à manipuler et à ranger.

Ces objets sont atteints en prévoyant que le tube de mesure soit amovible et puisse être retiré et remis en place de manière particulièrement simple.

La présente invention vise plus particulièrement un appareil de mesure de débit expiratoire comprenant un tube de mesure partiellement obturé par un rétrécissement de l'intérieur du tube, et un capteur de pression différentiel couplé au tube pour mesurer les pressions dans le tube de part et d'autre du rétrécissement. Le tube de mesure est amovible et comporte sur sa surface externe des joints annulaires souples dépassant de la surface externe et disposés de part et d'autre de lumières débouchant de part et d'autre du rétrécissement. Le tube de mesure est destiné à pénétrer dans un logement de l'appareil, de diamètre légèrement supérieur au diamètre extérieur du tube de mesure de manière que, dans une position en butée du tube de mesure dans le logement, les joints définissent avec le logement et la paroi externe du tube des chambres annulaires communiquant respectivement avec deux passages du logement, reliés au capteur de pression.

Selon un mode de réalisation de la présente invention, le logement est conique et la paroi externe du tube de mesure est complémentaire au logement par échelons, une transition d'un échelon à un autre s'effectuant à chaque joint.

Selon un mode de réalisation de la présente invention, les joints sont des joints toriques disposés dans des gorges du tube de mesure.

Selon un mode de réalisation de la présente invention, le logement comporte, de son côté de plus faible diamètre, un épaulement intérieur servant de butée et de centrage à l'extrémité de plus faible diamètre du tube de mesure.

Selon un mode de réalisation de la présente invention, l'appareil comprend un boîtier allongé dont l'axe longitudinal est parallèle à l'axe du tube de mesure.

Selon un mode de réalisation de la présente invention, l'extrémité interne du tube de mesure communique avec un pavillon pour dévier le flux d'air du tube vers un côté.

Ces objets, caractéristiques et avantages ainsi que d'autres de la présente invention seront exposés en détail dans la description suivante de modes de réalisation particuliers faite, à titre non-limitatif, en relation avec les figures jointes, parmi lesquelles :
la figure 1 représente un mode de réalisation de tube de mesure de débit amovible selon la présente invention ainsi que, de manière très schématique, des circuits électroniques associés ; et
les figures 2A et 2B représentent une vue de face et une vue de côté en coupe d'un mode de réalisation d'ensemble de spiromètre portable selon la présente invention.

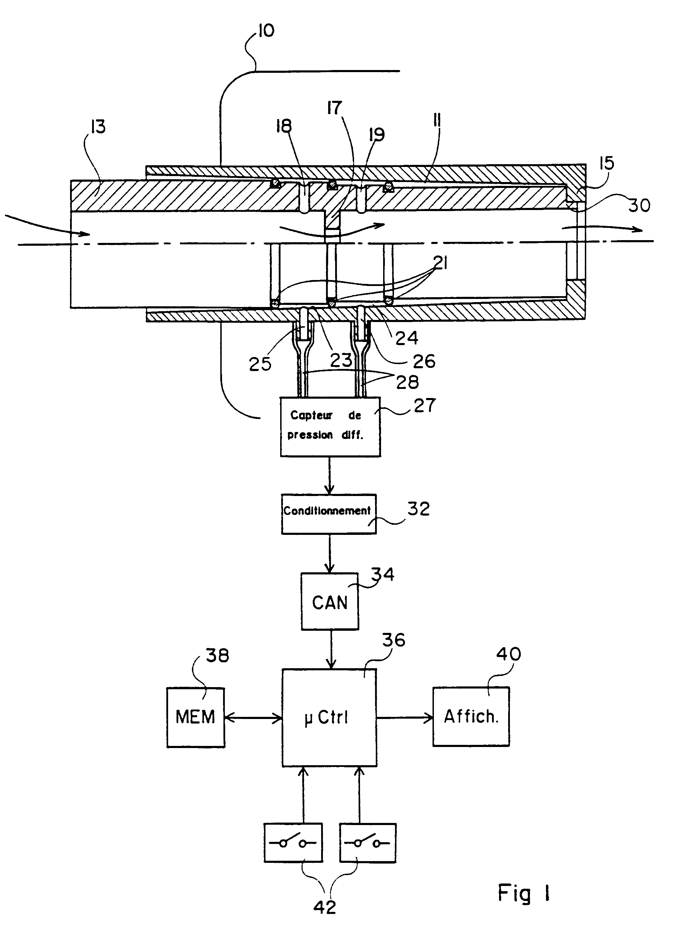

La figure 1 représente partiellement un boîtier 10 de spiromètre muni, selon l'invention, d'un logement cylindrique 11 dont les deux extrémités sont ouvertes sur l'atmosphère. Ce logement 11 est par exemple constitué de la paroi interne d'un tube solidaire du boîtier 10. A l'intérieur du logement 11 est disposé un tube de mesure amovible 13 de diamètre extérieur légèrement inférieur au diamètre du logement 11.

Le tube de mesure 13 est représenté dans sa position de mesure. L'une de ses extrémités (gauche) fait saillie en dehors du logement 11 et constitue un embout par lequel le patient souffle. L'autre extrémité du tube 13 est en butée contre un épaulement intérieur 15 du logement 11. Cet épaulement 15 peut être situé à convenance dans le boîtier 10.

Le tube de mesure 13 est ouvert à ses deux extrémités de manière que l'air soufflé par le patient puisse s'écouler librement d'une extrémité vers l'autre du tube de mesure 13, comme cela est représenté par des flèches. Le tube de mesure 13 est un tube de mesure à diaphgrame, ou Venturi. Le diamètre intérieur du tube 13 est sensiblement constant et comporte un rétrécissement local (ou diaphgrame) 17 à proximité du centre du tube. La paroi du tube 13 comporte deux lumières 18, 19 de part et d'autre du rétrécissement 17. La position relative de ces lumières 18, 19 importe peu et il peut y en avoir plusieurs de chaque côté du rétrécissement 17.

Entre les deux lumières 18, 19 et de part et d'autre de ces lumières sont disposés des joints toriques 21 dans des gorges de la surface externe du tube 13. Ces joints toriques 21 dépassent légèrement de la surface externe du tube 13 et viennent en contact avec la paroi du logement 11 de manière à délimiter, avec la paroi du logement 11 et la paroi externe du tube 13, deux chambres annulaires 23, 24 communiquant respectivement avec les lumières 18, 19. Le logement 11 est muni de deux passages 25, 26 débouchant respectivement dans chacune des deux chambres annulaires 23, 24 et couplés à un capteur de pression différentiel 27 par des tuyaux souples 28 ou une prise directe.

Avec cette configuration, les espaces de part et d'autre du rétrécissement 17 communiquent de manière étanche avec le capteur de pression 27 par l'intermédiaire des lumières 18, 19, des chambres annulaires 23, 24 et finalement par les passages 25, 26. Les chambres 23, 24 ont une faible section mais permettent néanmoins, quelle que soit la position des lumières 18, 19 par rapport aux passages 25, 26 de transmettre la pression de l'intérieur du tube 13 vers le capteur 27 de manière fiable car il n'y a aucun débit d'air du tube vers le capteur.

Dans la figure 1, les lumières 18, 19 ont été représentées du côté opposé des passages 25, 26, mais elles peuvent, bien entendu, être dans n'importe quelle position radiale, pourvu que leur position axiale soit telle que les passages 25, 26 communiquent avec leurs chambres respectives 23, 24.

Le tube 13 est maintenu dans le logement 11 par simple adhérence des joints 21. Ainsi, le tube 13 peut être extrait et remis en place sans dispositif de maintien particulier et sans précaution particulière, sauf à venir pousser le tube 13 en butée contre l'épaulement 15 qui définit le bon positionnement des chambres 23, 24 par rapport aux passages 25, 26 reliés au capteur de pression. Ceci permet une manipulation particulièrement rapide et aisée du tube de mesure 13.

Lorsqu'un patient souffle dans le tube 13, sa salive et d'éventuelles expectorations viennent adhérer à la paroi intérieure du tube 13. La salive peut éventuellement pénétrer dans les lumières 18, 19, mais il est très peu probable qu'elle atteigne la paroi du logement 11, car le débit dans les lumières 18, 19 est nul. Ainsi, pour nettoyer et stériliser efficacement l'appareil, il suffit de retirer le tube 13 et de le laver ou de le tremper dans une solution antiseptique. Il devient inutile de laver l'appareil entier pour garantir des conditions d'hygiène satisfaisantes. Il est donc inutile que l'appareil soit étanche.

Le fait que le tube de mesure 13 soit amovible permet au médecin de disposer de plusieurs tubes pour effectuer des mesures sur des patients successifs. Les tubes 13 sont des pièces simples, donc peu coûteuses, que l'on peut fabriquer en matière plastique moulée.

Selon un mode de réalisation, la paroi du logement 11 est légèrement conique, le plus petit diamètre étant du côté de la butée 15. Ceci permet, la paroi externe du tube 13 étant complémentaire, de retirer et engager le tube 13 dans le logement 11 avec un faible effort. En effet, les joints toriques 21 n'entrent alors en frottement avec le logement 11 que sur une faible distance lors de l'insertion et du retrait du tube 13. L'extrémité intérieure du tube 13 peut comporter, comme cela est représenté, un épaulement de centrage 30 coopérant avec l'épaulement de butée 15.

Selon un autre mode de réalisation, le logement 11 étant toujours conique, la surface externe du tube 13 peut être réalisée, comme cela est représenté, par des parties cylindriques de diamètres échelonnés de manière à approcher une conicité complémentaire de celle du logement 11. Chaque transition de diamètre s'effectue au niveau de l'un des joints toriques 21. Cette solution permet de simplifier la fabrication du tube 13.

Un circuit électronique, disposé dans le boîtier 10, permettant d'exploiter le signal du capteur 27 est représenté de façon très schématique à titre d'exemple à la figure 1. Le capteur différentiel 27 peut être un capteur différentiel commercialement disponible, tel que celui commercialisé sous l'appellation MPX10D par la société Motorola. Le signal fourni par ce capteur 27 est traité par un circuit de conditionnement 32 (amplificateur, filtre, intégrateur, etc.) avant d'arriver à un convertisseur analogique/numérique 34. Le signal numérique fourni par le convertisseur 34 est exploité par un microcontrôleur 36 qui peut stocker la valeur du débit dans une mémoire 38 et l'afficher sur un afficheur 40. Des boutons poussoirs 42 servent à choisir plusieurs fonctions du microcontrôleur 36 autres que la simple mesure de débit, par exemple, l'affichage de débits précédemment mesurés ainsi que la date et l'heure de ces mesures.

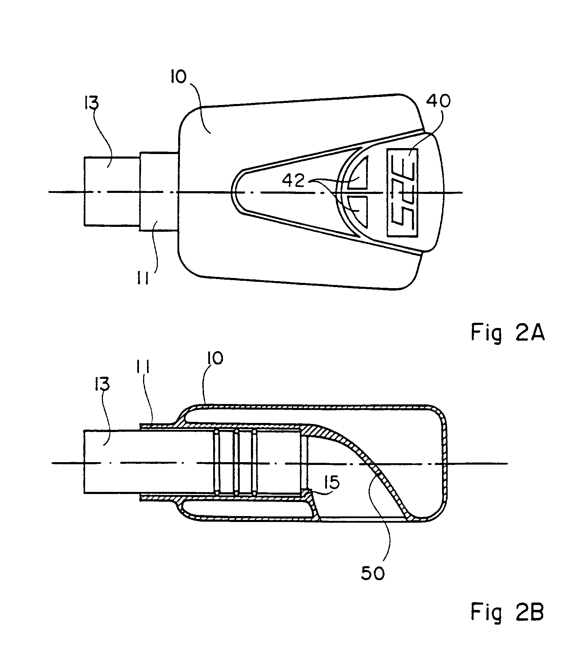

Les figures 2A et 2B représentent respectivement une vue de face et une vue de côté en coupe d'un mode de réalisation d'ensemble de spiromètre portable selon l'invention.

A la figure 2A, le boîtier 10 de l'appareil est de forme trapézoïdale à coins arrondis. Le tube 13 fait saillie du petit côté du trapèze et le logement 11 est formé par un tube solidaire du boîtier 10. L'afficheur 40 se trouve à proximité du grand côté du trapèze et est disposé parallèlement à celui-ci. Les boutons 42 sont disposés côte à côte en dessous de l'afficheur 40.

A la figure 2B, le logement 11, du côté de l'épaulement 15, est prolongé par un pavillon 50, solidaire du boîtier 10, qui dévie le flux d'air vers une ouverture de la face inférieure du boîtier 10, c'est-à-dire perpendiculairement à l'axe du tube 13.

Avec cette configuration, l'appareil est particulièrement peu encombrant et peut facilement se ranger dans une poche, notamment grâce au fait qu'aucune pièce allongée ne fait saillie perpendiculairement à l'axe longitudinal du boîtier 10. Le logement 11 et le pavillon 50 sont faciles à nettoyer, le cas échéant, à l'aide d'un chiffon ou un coton imbibé de solution antiseptique. En pratique, un tel nettoyage a lieu beaucoup moins souvent que le nettoyage du tube de mesure 13.

Le boîtier 10, intégrant le logement 11 et le pavillon 50, est facile à réaliser en une seule opération de moulage de matière plastique.

Bien entendu, la présente invention est susceptible de diverses variantes et modifications qui apparaîtront à l'homme de l'art et le domaine de protection est défini par la portée des revendications ci-après.

Un premier exemple de variante concerne les joints entre le tube de mesure et son logement. Au lieu de joints toriques rapportés dans des gorges du tube de mesure, on pourra prévoir que ces joints sont des collerettes faisant partie intégrante du tube. Cette variante s'applique en particulier à la réalisation de tubes jetables en matière plastique.

Un autre exemple de variante concerne la butée entre le tube de mesure et son logement. Au lieu d'un épaulement intérieur au fond du logement, on pourra prévoir un épaulement extérieur sur le tube de mesure, à proximité de son embouchure.

## Revendications

1. Appareil de mesure de débit expiratoire comprenant :
- un tube de mesure (13) partiellement obturé par un rétrécissement (17) de l'intérieur du tube ;
- un capteur de pression différentiel (27) couplé au tube pour mesurer les pressions dans le tube de part et d'autre du rétrécissement ;
caractérisé en ce que le tube de mesure (13) est amovible et comporte sur sa surface externe des joints annulaires souples (21) dépassant de la surface externe et disposés de part et d'autre de lumières (18, 19) débouchant de part et d'autre du rétrécissement, le tube de mesure étant destiné à pénétrer dans un logement (11) de l'appareil, de diamètre légèrement supérieur au diamètre extérieur du tube de mesure de manière que, dans une position en butée (15) du tube de mesure dans le logement, les joints définissent avec le logement (11) et la paroi externe du tube (13) des chambres annulaires (23, 24) communiquant respectivement avec deux passages (25, 26) du logement, reliés au capteur de pression.

2. Appareil selon la revendication 1, caractérisé en ce que le logement (11) est conique et la paroi externe du tube de mesure (13) est complémentaire au logement par échelons, une transition d'un échelon à un autre s'effectuant à chaque joint (21).

3. Appareil selon la revendication 1, caractérisé en ce que les joints sont des joints toriques disposés dans des gorges du tube de mesure.

4. Appareil selon la revendication 2, caractérisé en ce que le logement (11) comporte, de son côté de plus faible diamètre, un épaulement intérieur (15) servant de butée et de centrage à l'extrémité (30) de plus faible diamètre du tube de mesure.

5. Appareil selon la revendication 1, caractérisé en ce qu'il comprend un boîtier allongé (10) dont l'axe longitudinal est parallèle à l'axe du tube de mesure (13).

6. Appareil selon la revendication 5, caractérisé en ce que l'extrémité interne du tube de mesure (13) communique avec un pavillon (50) pour dévier le flux d'air du tube vers un côté.

7. Appareil selon la revendication 1, caractérisé en ce que les joints sont des collerettes faisant partie intégrante du tube de mesure.

## Claims

1. A device for measuring exhaled flow comprising:
- a measuring tube (13) partially obstructed by a narrow part (17) of the tube;
- a differential pressure sensor (27) coupled to the tube for measuring the pressures inside the tube on either side of the narrow part;
characterized in that the measuring tube (13) is removable and comprises, at its outer surface, flexible annular seals (21) protruding from the outer surface and located on either side of openings (18, 19) in turn located on either side of the narrow part, the measuring tube being insertable in a recess (11) of said device, said recess having a diameter slightly greater than the external diameter of the measuring tube such that, in an abutment position (15) of the measuring tube in the recess, the annular seals define with the recess (11) and the external wall of the tube (13) annular chambers (23, 24) communicating respectively with two passages (25, 26) of the recess, connected to the pressure sensor.

2. A device according to claim 1, characterized in that the recess (11) is tapered and the external wall of the measuring tube (13) is complementary to the recess by steps, a step transition being situated at the level of each annular seal (21).

3. A device according to claim 1, characterized in that the seals are O-rings placed in grooves of the measuring tube.

4. A device according to claim 2, characterized in that the recess (11) comprises, at its end of smallest diameter, an internal shoulder (15) for stopping and centering the end (30) of smallest diameter of the measuring tube.

5. A device according to claim 1, characterized in that it comprises an elongated casing (10) whose longitudinal axis is parallel to the axis of the measuring tube (13).

6. A device according to claim 5, characterized in that the internal end of the measuring tube (13) communicates with a horn (50) for deviating the air flow in the tube towards a side.

7. A device according to claim 1, characterized in that the annular seals are flanges integral with the measuring tube.

## Patentansprüche

1. Vorrichtung zum Messen einer ausgeatmeten Strömung, mit
einem Meßrohr (13), das durch eine Verengung (17) des Rohres teilweise behindert ist;
einem Differenzdrucksensor (27), der mit dem Rohr gekoppelt ist, um den Druck innerhalb des Rohres auf beiden Seiten der Verengung zu messen;
dadurch **gekennzeichnet**, daß das Meßrohr (13) entfernbar ist und an seiner Außenseite flexible ringförmige Dichtungen (21) aufweist, die von der Außenseite vorstehen und auf beiden Seiten von Öffnungen (18, 19) angeordnet sind, welche ihrerseits auf beiden Seiten der Verengung angeordnet sind, wobei das Meßrohr in eine Aufnahme (11) der Vorrichtung einsetzbar ist, die Aufnahme einen Durchmesser hat, der geringfügig größer als der Außenmesser des Meßrohres ist, so daß dann, wenn das Meßrohr in der Aufnahme (15) sitzt, die ringförmigen Dichtungen mit der Aufnahme (11) und der Außenwand des Rohres (13) ringförmige Kammern (23, 24) definieren, welche jeweils mit zwei Durchgängen (25, 26) der Aufnahme, die mit dem Drucksensor verbunden sind, in Verbindung stehen.

2. Vorrichtung nach Anspruch 1, dadurch **gekennzeichnet**, daß die Aufnahme (11) abgeschrägt ist und die Außenwand des Meßrohres (13) stufenweise komplementär zu der Aufnahme ist, wobei ein Stufenübergang auf dem Niveau jeder ringförmigen Dichtung (21) liegt.

3. Vorrichtung nach Anspruch 1, dadurch **gekennzeichnet**, daß die Dichtungen O-Ringe sind, welche in Rillen des Meßrohres angeordnet sind.

4. Vorrichtung nach Anspruch 2, dadurch **gekennzeichnet**, daß die Aufnahme (11) an ihrem Ende mit dem kleinsten Durchmesser eine Innenschulter (15) aufweist, um das Ende (30) des kleinsten Durchmessers des Meßrohres zu stoppen und zu zentrieren.

5. Vorrichtung nach Anspruch 1, dadurch **gekennzeichnet**, daß sie ein langgestrecktes Gehäuse (10) aufweist, dessen Längsachse parallel zu der Achse des Meßrohres (13) ist.

6. Vorrichtung nach Anspruch 5, dadurch **gekennzeichnet**, daß das innere Ende des Meßrohres (13) mit einem Horn (50) in Verbindung steht, um die Luftströmung in dem Rohr zur Seite abzulenken.

7. Vorrichtung nach Anspruch 1, dadurch **gekennzeichnet**, daß die ringförmigen Dichtungen einstückig mit dem Meßrohr ausgebildete Flansche sind.
